# EUROPEAN PATENT SPECIFICATION

(11) **EP 3 020 809 B1**
(45) Date of publication and mention of the grant of the patent: **04.09.2019**
(21) Application number: 14823515.3
(22) Date of filing: 10.07.2014
(51) Int. Cl.: C12N 15/09

(54) **COMPOSITION FOR TRANSFERRING GENE TO CELL**
ZUSAMMENSETZUNG ZUM GENTRANSFER IN ZELLEN
COMPOSITION POUR TRANSFÉRER UN GÈNE VERS UNE CELLULE

(30) Priority: 12.07.2013 JP 2013146742; 29.11.2013 JP 2013248324
(43) Date of publication of application: 18.05.2016
(73) Proprietor: Ishihara Sangyo Kaisha, Ltd., Osaka-shi, Osaka 550-0002 (JP)
(72) Inventor: KONDO, Yoshitaka, Kusatsu-shi Shiga 525-0025 (JP)
(74) Representative: Arends, William Gerrit
(86) International application number: PCT/JP2014/068439
(87) International publication number: WO 2015/005431

(56) References cited:
- WO-A1-01/92508
- WO-A1-02/31138
- WO-A1-2009/028421
- WO-A1-2010/004989
- WO-A2-2013/032643
- JP-A- 2012 060 997
- OHAMA, Y. ET AL.: 'Gene Transfection into Hela Cells by Vesicles Containing Cationic Peptide Lipid' BIOSCI. BIOTECHNOL. BIOCHEM. vol. 69, no. 8, 2005, pages 1453 - 1458, XP055311062
- LIU, F. ET AL.: 'Effect of Non-Ionic Surfactants on the Formation of DNA/Emulsion Complexes and Emulsion-Mediated Gene Transfer' PHARMACEUTICAL RESEARCH vol. 13, no. 11, 1996, pages 1642 - 1646, XP001012538
- YOSHITAKA KONDO ET AL.: 'Shinki Hi-Virus-kei Idenshi Donyu Vector' ANNUAL MEETING OF THE MOLECULAR BIOLOGY SOCIETY OF JAPAN PROGRAM YOSHISHU (WEB) vol. 36 TH, no. #1P-09, 20 November 2013, XP008182726

## Description

### Technical Field

The present invention relates to a composition for introducing a gene into cells, a method for introducing a gene into cells, and a kit for introducing a gene into cells.

### Background Art

Gene transfer into cells is now a dominant technique in a wide variety of fields, such as molecular biology, cell biology, and genetic engineering. The methods for introducing a gene into cells can be broadly classified into two categories.

One is a method using a viral vector. The method using a viral vector has high transfer efficiency, but often poses safety problems.

The other is a method using no viral vector. A specific known example of this method is one in which plasmid DNA or the like is incorporated in, for example, a liposome, polyethyleneimine, a virus-derived envelope, or the like (carrier).

Examples of known methods for preparing liposomes include the lower-alcohol method, the Bangham method (thin-film method), and the like. In the lower-alcohol method, after a lipid is dissolved in a lower alcohol, such as methanol or ethanol, the resulting lipid solution is mixed with a buffer or the like and stirred, thereby preparing a liposome-containing suspension. In the Bangham method, after a lipid is dissolved in an organic solvent, such as chloroform or a chloroform/methanol mixed solvent, and the organic solvent is volatilized to form a thin lipid film, the film is mixed with a buffer or the like and stirred, thereby preparing a liposome-containing suspension. Other than these methods, it has been reported that liposomes for gene transfer were prepared by the SUV method (Non-patent Literature 1) or by the two-stage emulsification method (Patent Literature 1 and 2).

A known gene transfer method using a virus-derived envelope is a method using a Sendai virus envelope. In this method, a gene transfer vector is prepared by fusing a liposome having a gene encapsulated in the liposome and a virus-derived envelope obtained by destroying genomic RNA of Sendai virus with β-propiolactone or by UV irradiation or the like and then performing purification (Non-patent Literature 2); or a gene transfer vector is prepared by treating a virus-derived envelope with a surfactant to temporarily improve membrane permeability and thereby encapsulate plasmid DNA in the virus-derived envelope (Patent Literature 3).

Moreover, also known is a method in which a gene is incorporated in a virus-like envelope reconstituted from an artificial lipid membrane and a virus-derived membrane protein (Non-patent Literature 3).

It has also been reported that foreign gene expression was successfully improved by modifying a membrane surface protein of an enveloped virus using genetic modification technology (Patent Literature 4), by using polyethyleneimine (Non-patent Literature 4), or by combining a cationic lipid and an extracellular matrix (Patent Literature 5).

In the reverse transfection method, in which a gene transfer vector is immobilized and brought into contact with cells, a method using a gene delivery material and sericin or a hydrolysate thereof in combination is also known (Patent Literature 6). With this method, the expression of a foreign gene with respect to adherent cells is expected to be improved; however, improvement in the expression of a foreign gene with respect to suspension cells is limited.

These gene transfer methods using no viral vector have improved safety but often have inferior gene expression efficiency compared with methods using viral vectors. In addition, as stated above, preparation of liposomes and immobilization of gene transfer vectors are complicated, and the preparation requires a great deal of time and effort.

### Citation List

### Patent Literature

PTL 1: JP2005-068120A
PTL 2: JP2003-001097A
PTL 3: JP2001-286282A
PTL 4: JP2011-050292A
PTL 5: WO2005/073385
PTL 6: WO2009/028421

### Non-patent Literature

NPL 1: Construction of a multifunctional envelope-type nano device by a SUV-fusion method. Int J Pharm. 2005 May 30; 296(1-2): 142-150. Epub Apr. 11, 2005
NPL 2: Gene therapy using HVJ-liposomes: the best of both worlds. Mol Med Today. July 1999; 5(7): 298-303
NPL 3: Effect of Lipid Compositions on Gene Transfer into 293 Cells Using Sendai F/HN-virosomes. J Biochem Mol Biol. Sept. 30, 2002; 35(5): 459-464
NPL 4: Nonviral gene delivery: principle, limitations, and recent progress. The AAPS Journal. Dec. 2009; 11(4): 671-681

### Summary of Invention

### Technical Problem

Although various gene transfer methods are currently used, there is demand for development of a method that further satisfactorily achieves convenience in use, safety, and transfer efficiency. In addition, since there are a wide variety of cells into which a gene is to be introduced, the existing methods may not necessarily be satisfactory.

### Solution to Problem

The present inventor conducted extensive research and found that a composition for introducing a foreign gene into cells, the composition having excellent safety and gene transfer efficiency, can be obtained in a simple operation in which specific substances are only mixed. The present invention has been thus accomplished.

Specifically, the present invention relates to (1) a composition for gene transfer, the composition comprising:
(A) at least one lipid (hereinafter also referred to as "component A") selected from the group consisting of sorbitan sesquioleate, sorbitan monolaurate, sorbitan monopalmitate, sorbitan monostearate, sorbitan trioleate, sorbitan monooleate, L-α-phosphatidylinositol, L-α-dioleoyl phosphatidylethanolamine, octadecylamine, hexadecylamine, DOTAP (1,2-dioleoyl-3-trimethylammonium-propane), and cardiolipin;
(B) at least one protein (hereinafter also referred to as "component B") selected from the group consisting of albumin, casein, gelatin, and sericin; and
(C) at least one positively charged substance (hereinafter also referred to as "component C") selected from the group consisting of protamine sulfate, polyarginine, polylysine, polyethyleneimine, and hexadimethrine bromide.

The present invention also relates to (2) the composition according to (1), further comprising a gene.

The present invention also relates to (3) the composition according to (1) or (2), further comprising (D) at least one extracellular matrix component (hereinafter also referred to as "component D") selected from the group consisting of laminin, fibronectin, vitronectin, hyaluronic acid, entactin, elastin, tenascin, collagen, chondroitin sulfate, fibrin, and fibrinogen.

The present invention also relates to (4) the composition according to any one of (1) to (3), further comprising (E) at least one member (hereinafter also referred to as "component E") selected from the group consisting of chloroquine, quinacrine, hydroxychloroquine, cyclosporin, cyclophosphamide, tacrolimus, ascomycin, rapamycin, 2-cyano-3-(3,4-dihydroxyphenyl)-N-benzylacrylamide, amlexanox, N-[3-[[5-iodo-4-[[3-[(2-thienylcarbonyl)amino]propyl]amino]-2-pyrimidinyl]amino]phenyl]-1-pyrrolidinecarboxamide, doxorubicin, actinomycin D, aurintricarboxylic acid, paclitaxel, etoposide, N-[1-(3-(5-chloro-3-methylbenzo[b]thiophen-2-yl)-1-methyl-1H-pyrazol-5-yl)]-2-chlorobenzenesulfonamide, α-amanitin, β-amanitin, amlodipine, nifedipine, nicardipine, verapamil, diltiazem, trichostatin A, tubastatin A, suberoyl bis-hydroxamic acid, suberoylanilide hydroxamic acid, valproic acid, and phorbol 12-myristate 13-acetate.

The present invention also relates to (5) the composition according to any one of (1) to (4), further comprising (F) a viral envelope (hereinafter also referred to as "component F").

The present invention also relates to (6), the composition according to (1), wherein component A is at least one member selected from the group consisting of sorbitan sesquioleate and sorbitan monolaurate.

The present invention also relates to (7), the composition according to (1), wherein component A is sorbitan sesquioleate.

The present invention also relates to (8) a composition for use in introducing a gene into a cell of a non-human animal in *in vivo* therapy, the composition comprising component A, component B, component C.

The present invention also relates to (9) a method for introducing a gene into a cell in vitro, the method comprising bringing component A, component B, component C, and the gene into contact with the cell, wherein component A, component B, component C, and the gene are brought into contact with the cell simultaneously.

The present invention also relates to (10) the method according to (8) or (9), wherein further component D is brought into contact with the cell.

The present invention also relates to (11) the method according to any one of (8) to (10), wherein further component E is brought into contact with the cell.

The present invention also relates to (12) the method according to any one of (8) to (11), wherein further component F is brought into contact with the cell.The present invention also relates to (13) a kit for gene transfer, the kit comprising component A, component B, and component C.

The present invention also relates to (14) the kit according to (13), further comprising component D.

The present invention also relates to (15) the kit according to (13) or (14), further comprising component E.

The present invention also relates to (16) the kit according to any one of (13) to (15), further comprising component F.

### Advantageous Effects of Invention

The present invention makes it possible to provide a composition for introducing a foreign gene into cells, the composition having excellent safety and gene transfer efficiency. The composition can be prepared in a simple operation in which specific substances are only mixed. Further, the composition is also advantageous in terms of costs compared with carriers or the like used in the existing methods.

### Brief Description of Drawings

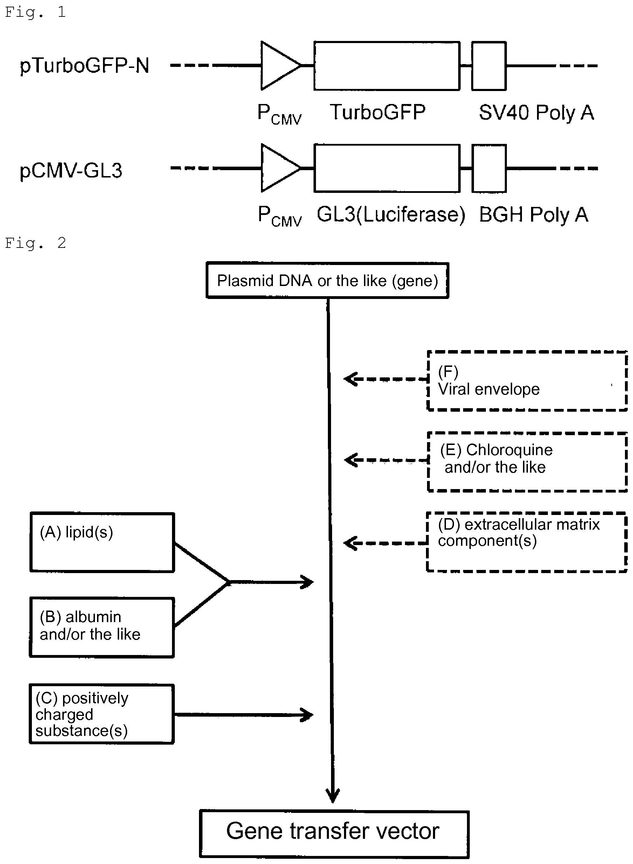

Fig. 1 shows plasmid DNA used in the experiments.
Fig. 2 shows an outline of the preparation method and illustrates an example of a procedure of adding each component to plasmid DNA. In Fig. 2, (A), (B), (C), (D), (E), and (F) indicate component A, component B, component C, component D, component E, and component F, respectively.

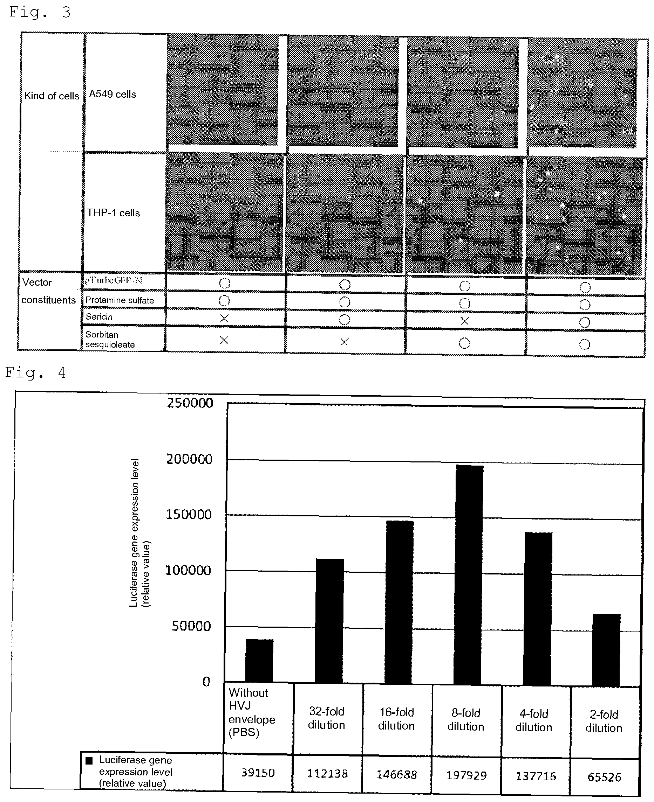

Fig. 3 is a fluorescence micrograph of cells into which pTurboGFP-N was introduced, one day after addition of a mixture containing plasmid DNA (pTurboGFP-N), (A) sorbitan sesquioleate (Span 83), (B) sericin, and (C) protamine sulfate to the cultured cells. In the "Vector constituents" row in Fig. 3, the circle indicates that the component is contained, whereas the X symbol indicates that the component is not contained.
Fig. 4 shows the expression level of luciferase gene measured one day after addition of a mixture containing plasmid DNA (pCMV-GL3), (F) an envelope derived from Sendai virus, (A) sorbitan sesquioleate, (B) sericin, and (C) protamine sulfate to cultured cells.

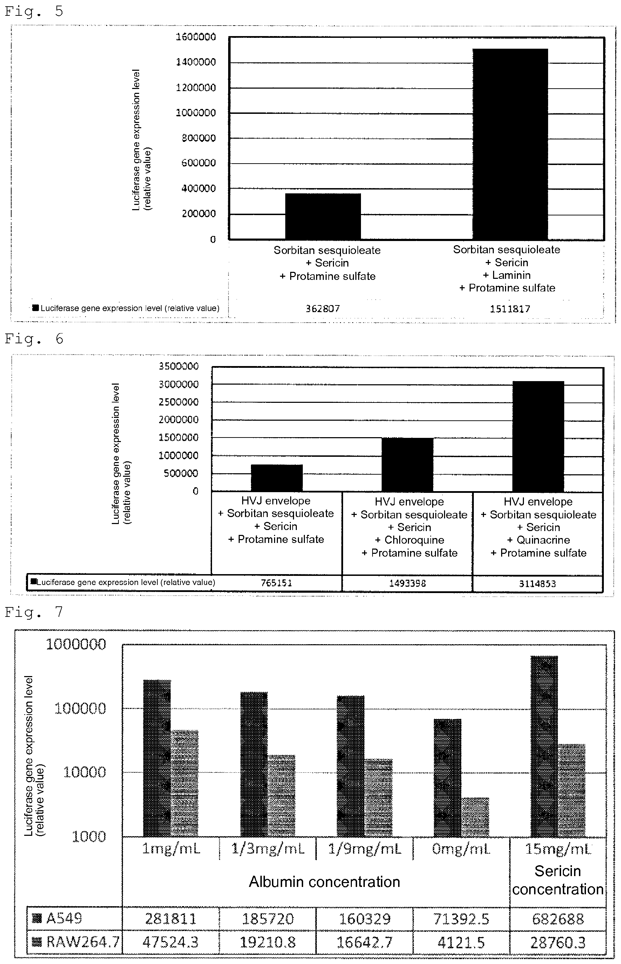

Fig. 5 shows the expression level of luciferase gene measured one day after addition of a mixture containing plasmid DNA (pCMV-GL3), (A) sorbitan sesquioleate, (B) sericin, (C) protamine sulfate, and (D) laminin to cultured cells. The bar on the left shows the expression level of luciferase gene obtained when (D) laminin was not added.
Fig. 6 shows the expression level of luciferase gene measured one day after addition of a mixture containing plasmid DNA (pCMV-GL3), (F) an envelope derived from Sendai virus, (A) sorbitan sesquioleate, (B) sericin, (C) protamine sulfate, and (E) chloroquine or quinacrine to cultured cells. The bars from left to right are as follows: the case in which component E was not added, the case in which chloroquine was added as component E, and the case in which quinacrine was added as component E.
Fig. 7 shows the expression level of luciferase gene measured one day after addition of a mixture containing plasmid DNA (pCMV-GL3), (F) an envelope derived from Sendai virus, (A) sorbitan sesquioleate, (B) albumin, and (C) protamine sulfate to cultured cells (the bars from left to right are as follows: albumin 1 mg/mL, 1/3 mg/mL, 1/9 mg/mL, and without albumin). Fig. 7 also shows the expression level of luciferase gene measured one day after addition of a mixture containing plasmid DNA (pCMV-GL3), (F) an envelope derived from Sendai virus, (A) sorbitan sesquioleate, (B) sericin, and (C) protamine sulfate to cultured cells (on the far right of the bar graph).

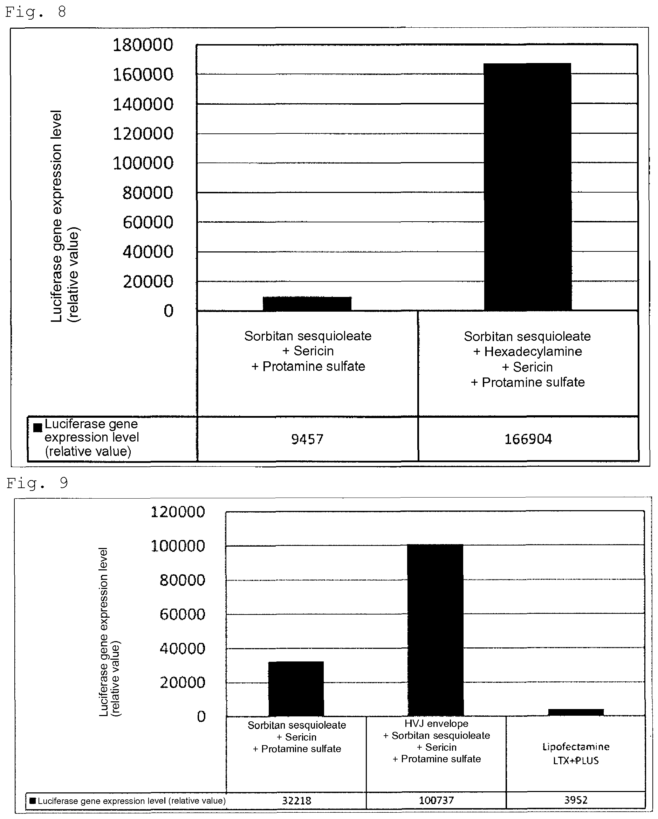

Fig. 8 shows the expression level of luciferase gene measured one day after addition of a mixture containing plasmid DNA (pCMV-GL3), (A) sorbitan sesquioleate, (A) hexadecylamine, (B) sericin, and (C) protamine sulfate to cultured cells. The bar on the left shows the expression level of luciferase gene obtained when (A) hexadecylamine was not added.
Fig. 9 shows a comparison in the gene expression level between mixture 1 containing plasmid DNA (pCMV-GL3), (A) sorbitan sesquioleate, (B) sericin, and (C) protamine sulfate; mixture 2 containing plasmid DNA (pCMV-GL3), (F) an envelope derived from Sendai virus, (A) sorbitan sesquioleate, (B) sericin, and (C) protamine sulfate; and a commercially available gene transfer reagent (comparative mixture). The bars from left to right are as follows: mixture 1, mixture 2, and the comparative mixture.

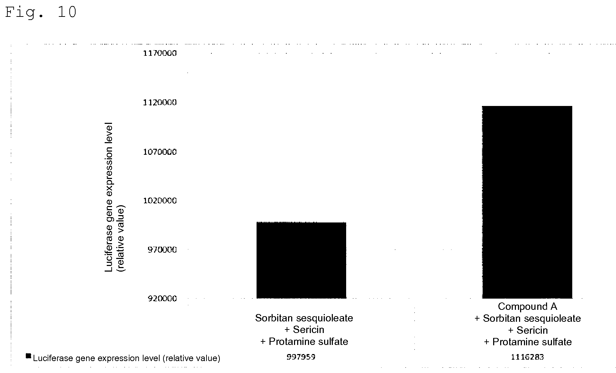

Fig. 10 shows the expression level of luciferase gene measured one day after addition of a mixture containing plasmid DNA (pCMV-GL3), (A) sorbitan sesquioleate, (B) sericin, (C) protamine sulfate, and (E) N-[3-[[5-iodo-4-[[3-[(2-thienylcarbonyl)amino]propyl]amino]-2-pyrimidinyl]amino]phenyl]-1-pyrrolidinecarboxamide (hereinafter also referred to as "compound A") to cultured cells. The bar on the left shows the expression level of luciferase gene obtained when (E) compound A was not added.

### Description of Embodiments

As used herein, "gene" refers to a naturally occurring, synthetic, or recombinant gene, or a gene fragment thereof; and "gene transfer" refers to introducing a desired naturally occurring, synthetic, or recombinant gene, or a gene fragment thereof into a target cell in vivo or in vitro in such a manner that the introduced gene maintains its function. The gene or gene fragment introduced in the present invention encompasses DNA or RNA having a specific sequence or a nucleic acid that is a synthetic analog thereof. The terms "gene transfer" and "transfection" as used herein are used interchangeably.

In the present specification, examples of cells into which a gene is introduced include in vitro cultured cells, cells isolated from organisms, and cells in vivo. The cells may be either adherent cells or suspension cells.

In the present specification, the meaning of "sericin" includes sericin and a hydrolysate thereof.

In the present specification, examples of viral envelopes include envelopes derived from viruses belonging to Retroviridae, Togaviridae, Coronaviridae, Flaviviridae, Paramyxoviridae, Orthomyxoviridae, Bunyaviridae, Rhabdoviridae, Poxviridae, Herpesviridae, Baculoviridae, and Hepadnaviridae. The viral envelope is preferably an envelope derived from Sendai virus (HVJ: hemagglutinating virus of Japan). The viral envelope is more preferably an envelope derived from inactivated Sendai virus (HVJ).

The phrases "method for introducing a gene into a cell of a non-human animal" and "method for introducing a gene into an in vitro cell" as used herein expressly do not include methods for treating humans.

Unlike complicated methods for preparing liposomes, the composition of the present invention can be prepared in a simple operation in which component A, component B, and component C are only mixed by, for example, pipetting or tapping, or using a vortex mixer. Moreover, improvement in gene expression efficiency can be expected by further, for example, mixing component D, component E, and/or component F to suitably prepare the composition of the present invention.

The weight ratio of component A to component B in the composition of the present invention is not particularly limited, and preferably 1:0.07 to 1:4200, more preferably 1:4 to 1:4200, even more preferably 1:4 to 1:1000, and still even more preferably 1:35 to 1:840.

The weight ratio of component A to component C cannot be generalized, because the time required for gene transfer varies depending on the amount of component C, and is preferably 1:0.006 to 1:320, more preferably 1:0.1 to 1:320, even more preferably 1:0.1 to 1:50, and still even more preferably 1:1.5 to 1:50.

When the composition of the present invention further comprises component D, the weight ratio of component A to component D is not particularly limited, and preferably 1:0.002 to 1:140, more preferably 1:0.04 to 1:140, even more preferably 1:0.04 to 1:70, and still even more preferably 1:0.1 to 1:10.

When the composition of the present invention further comprises component E, the weight ratio of component A to component E cannot be generalized, because it varies depending on the type of component E, and may be selected as desired in a range that does not inflict toxicity upon cells into which a gene is introduced.

When the composition of the present invention further comprises component F, in particular, when it comprises an envelope derived from Sendai virus as component F, the ratio of component A to the envelope derived from Sendai virus (HAU of the the envelope derived from Sendai virus relative to 1 µg of component A) is not particularly limited, and preferably 1:0.01 to 1:3000, more preferably 1:0.1 to 1:3000, even more preferably 1:0.3 to 1:500, and still even more preferably 1:0.5 to 1:100.

The concentration of component A in the composition of the present invention is not particularly limited, and preferably 0.0003 to 0.2% by weight, more preferably 0.0003 to 0.02% by weight, even more preferably 0.0005 to 0.02% by weight, and still even more preferably 0.0007 to 0.001% by weight.

The concentration of component B in the composition of the present invention is not particularly limited, and preferably 0.01 to 3% by weight, more preferably 0.04 to 2% by weight, even more preferably 0.04 to 1% by weight, and still even more preferably 0.2 to 1% by weight.

The concentration of component C in the composition of the present invention cannot be generalized, because the time required for gene transfer varies depending on the amount of component C, and is preferably 0.0009 to 0.13% by weight and more preferably 0.009 to 0.06% by weight.

When the composition of the present invention further comprises component D, the concentration of component D is not particularly limited, and preferably 0.0003 to 0.04% by weight and more preferably 0.0008 to 0.004% by weight.

When the composition of the present invention further comprises component E, the concentration of component E cannot be generalized, because it varies depending on the type of component E, and may be selected as desired in a range that does not inflict toxicity upon cells into which a gene is introduced.

When the composition of the present invention further comprises component F, in particular, when it comprises an envelope derived from Sendai virus as component F, HAU of the envelope derived from Sendai virus per unit of amount of liquid is not particularly limited, and preferably 0.01 to 10 HAU/µL and more preferably 0.03 to 1 HAU/µL.

The composition of the present invention may comprise not only component A, component B, and component C, and optionally component D, component E, and/or component F, but also additive(s), such as water, alcohols (e.g., ethanol), buffers (e.g., phosphate buffered saline, HEPES buffer, Tris-HCl buffer, TE buffer), and cell culture media (e.g., DMEM medium, RPMI medium). The concentration of these additives is not particularly limited. The composition of the present invention preferably has a pH of 6 to 10.

The present invention also relates to a composition for use in introducing a gene into a cell of a non-human animal in *in vivo* therapy by bringing component A, component B, component C, and the gene into contact with the cell, and a method for introducing a gene into a cell in vitro by bringing component A, component B, component C, and the gene into contact with the cell simultaneously. In the composition for use and the method of the present invention, improvement in gene transfer efficiency can be expected by bringing further component D, component E, and/or component F into contact with the cell.

The method of the present invention can be performed under freely selected conditions.

It is particularly preferred that a gene transfer vector comprising a gene, component A, component B, and component C, and optionally component D, component E, and/or component F be prepared and brought into contact with cells. In the present invention, some or all of the components and the gene may be in the form of a complex. The term "gene transfer vector" as used herein does not mean a single nucleic acid, but means one in the form of a composition containing not only a nucleic acid, but also component(s) other than the nucleic acid (e.g., component A).

The gene transfer vector can be prepared by mixing each component and a gene in any mixing order by, for example, pipetting. Specific examples include (i) mixing a gene to be introduced with a mixture of component A, component B, and component C; (ii) mixing a gene to be introduced with a mixture of component A and component B, and subsequently mixing the resulting mixture with component C; (iii) mixing a gene to be introduced with component C, and subsequently mixing the resulting mixture with a mixture of component A and component B; (iv) mixing a gene to be introduced with component A, subsequently mixing the resulting mixture with component B, and lastly mixing the obtained mixture with component C; (v) mixing a gene to be introduced with component B, subsequently mixing the resulting mixture with component A, and lastly mixing the obtained mixture with component C; (vi) mixing a gene to be introduced with component B, subsequently mixing the resulting mixture with component C, and lastly mixing the obtained mixture with component A.

Even when component D, component E, and/or component F is further used, the gene transfer vector can be prepared by mixing each component in any mixing order by, for example, pipetting. Specific examples when component F is further used include (vii) mixing a gene to be introduced with component F and subsequently mixing the resulting mixture with a mixture of component A, component B, and component C; (viii) mixing a gene to be introduced with component F, subsequently mixing the resulting mixture with a mixture of component A and component B, and lastly mixing the obtained mixture with component C; (ix) mixing a gene to be introduced with a mixture of component A and component B, subsequently mixing the resulting mixture with component F, and lastly mixing the obtained mixture with component C; (x) mixing a gene to be introduced with a mixture of component A and component B, subsequently mixing the resulting mixture with component C, and lastly mixing the obtained mixture with component F.

Although there is no limitation on the mixing order in the preparation of the gene transfer vector, the gene transfer vector is preferably prepared by using the procedure shown in Fig. 2.

The ratio and concentration of the components in the method of the present invention are, for example, the same as the weight ratio and concentration mentioned above for the components in the composition of the present invention. The gene transfer vector may comprise additive(s) that are the same as the additives mentioned above for the composition of the present invention.

The kit for gene transfer of the present invention comprises component A, component B, and component C and may, if necessary, comprise component D, component E, and/or component F. The kit of the present invention may further comprise alcohols (e.g., ethanol), buffers (e.g., phosphate buffered saline, HEPES buffer, Tris-HCl buffer, TE buffer), cell culture media (e.g., DMEM medium, RPMI medium), etc.

The components of the kit of the present invention may be contained in separate containers, or any two or more of the components may be contained in a single container.

The kit for gene transfer of the present invention makes it possible to easily prepare the composition for gene transfer and gene transfer vector of the present invention.

Examples of preferred embodiments of the present invention are described below.
(1) A composition for gene transfer, the composition comprising;
   (A) at least one lipid selected from the group consisting of sorbitan sesquioleate, sorbitan monolaurate, sorbitan monopalmitate, sorbitan monostearate, sorbitan trioleate, sorbitan monooleate, L-α-phosphatidylinositol, L-α-dioleoyl phosphatidylethanolamine, octadecylamine, hexadecylamine, DOTAP, and cardiolipin;
   (B) at least one protein selected from the group consisting of albumin, casein, gelatin, and sericin; and
   (C) at least one positively charged substance selected from the group consisting of protamine sulfate, polyarginine, polylysine, polyethyleneimine, polyethyleneimine, and hexadimethrine bromide.
(2) The composition according to (1), further comprising (D) at least one extracellular matrix component selected from the group consisting of laminin, fibronectin, hyaluronic acid, entactin, elastin, tenascin, vitronectin, collagen, chondroitin sulfate, fibrin, and fibrinogen.
(3) The composition according to (1) or (2), further comprising (E) at least one member selected from the group consisting of chloroquine, quinacrine, hydroxychloroquine, cyclosporin, cyclophosphamide, tacrolimus, ascomycin, rapamycin, 2-cyano-3-(3,4-dihydroxyphenyl)-N-benzylacrylamide, amlexanox, N-[3-[[5-iodo-4-[[3-[(2-thienylcarbonyl)amino]propyl]amino]-2-pyrimidinyl]amino]phenyl]-1-pyrrolidinecarboxamide, doxorubicin, actinomycin D, aurintricarboxylic acid, paclitaxel, etoposide, N-[1-(3-(5-chloro-3-methylbenzo[b]thiophen-2-yl)-1-methyl-1H-pyrazol-5-yl)]-2-chlorobenzenesulfonamide, α-amanitin, β-amanitin, amlodipine, nifedipine, nicardipine, verapamil, diltiazem, trichostatin A, tubastatin A, suberoyl bis-hydroxamic acid, suberoylanilide hydroxamic acid, valproic acid, and phorbol 12-myristate 13-acetate.
(4) The composition according to any one of (1) to (3), further comprising (F) a viral envelope.
(5) The composition according to (1), wherein the lipid of (A) is at least one member selected from the group consisting of sorbitan sesquioleate, sorbitan monolaurate, and hexadecylamine.
(6) The composition according to (1), wherein the lipid of (A) is sorbitan sesquioleate.
(7) The composition according to (1), wherein the positively charged substance of (C) is protamine sulfate.
(8) The composition according to (2), wherein the extracellular matrix component of (D) is at least one member selected from the group consisting of laminin, vitronectin, and fibrinogen.
(9) The composition according to (4), wherein the viral envelope of (F) is an envelope derived from Sendai virus.
(10) A composition for use in introducing a gene into a cell of a non-human animal in *in vivo* therapy, the composition comprising:(A) at least one lipid selected from the group consisting of sorbitan sesquioleate, sorbitan monolaurate, sorbitan monopalmitate, sorbitan monostearate, sorbitan trioleate, sorbitan monooleate, L-α-phosphatidylinositol, L-α-dioleoyl phosphatidylethanolamine, octadecylamine, hexadecylamine, DOTAP, and cardiolipin; (B) at least one protein selected from the group consisting of albumin, casein, gelatin, and sericin; (C) at least one positively charged substance selected from the group consisting of protamine sulfate, polyarginine, polylysine, polyethyleneimine, polyethyleneimine, and hexadimethrine bromide; and the gene.
(11) A method for introducing a gene into a cell in vitro, the method comprising bringing, into contact with the cell, (A) at least one lipid selected from the group consisting of sorbitan sesquioleate, sorbitan monolaurate, sorbitan monopalmitate, sorbitan monostearate, sorbitan trioleate, sorbitan monooleate, L-α-phosphatidylinositol, L-α-dioleoyl phosphatidylethanolamine, octadecylamine, hexadecylamine, DOTAP, and cardiolipin; (B) at least one protein selected from the group consisting of albumin, casein, gelatin, and sericin; (C) at least one positively charged substance selected from the group consisting of protamine sulfate, polyarginine, polylysine, polyethyleneimine, polyethyleneimine, and hexadimethrine bromide; and the gene, wherein the lipid, the protein, the positively charged substance, and the gene are all brought into contact with the cell simultaneously.
(12) The composition for use according to (10) or method according to (11), wherein the composition further comprises (D) at least one extracellular matrix component selected from the group consisting of laminin, fibronectin, vitronectin, hyaluronic acid, entactin, elastin, tenascin, collagen, chondroitin sulfate, fibrin, and fibrinogen, or wherein the method further comprises bringing (D) into contact with the cell.
(13) The composition for use or method according to any one of (10) to (12), wherein the composition further comprises (E) at least one member selected from the group consisting of chloroquine, quinacrine, hydroxychloroquine, cyclosporin, cyclophosphamide, tacrolimus, ascomycin, rapamycin, 2-cyano-3-(3,4-dihydroxyphenyl)-N-benzylacrylamide, amlexanox, N-[3-[[5-iodo-4-[[3-[(2-thienylcarbonyl)amino]propyl]amino]-2-pyrimidinyl]amino]phenyl]-1-pyrrolidinecarboxamide, doxorubicin, actinomycin D, aurintricarboxylic acid, paclitaxel, etoposide, N-[1-(3-(5-chloro-3-methylbenzo[b]thiophen-2-yl)-1-methyl-1H-pyrazol-5-yl)]-2-chlorobenzenesulfonamide, α-amanitin, β-amanitin, amlodipine, nifedipine, nicardipine, verapamil, diltiazem, trichostatin A, tubastatin A, suberoyl bis-hydroxamic acid, suberoylanilide hydroxamic acid, valproic acid, and phorbol 12-myristate 13-acetate, or wherein the method further comprises bringing (E) into contact with the cell.
(14) The composition for use or method according to any one of (10) to (13), wherein the composition further comprises (F) a viral envelope, or wherein the method further comprises bringing (F) into contact with the cell.
(15) The composition for use or method according to any one of (10) to (14), wherein the lipid of (A) is at least one member selected from the group consisting of sorbitan sesquioleate, sorbitan monolaurate, and hexadecylamine.
(16) The composition for use or method according to any one of (10) to (14), wherein the lipid of (A) is sorbitan sesquioleate.
(17) The composition for use or method according to any one of (10) to (16), wherein the positively charged substance of (C) is protamine sulfate.
(18) The composition for use or method according to any one of (12) to (17), wherein the extracellular matrix component of (D) is at least one member selected from the group consisting of laminin, vitronectin, and fibrinogen.
(19) The composition for use or method according to any one of (14) to (18), wherein the viral envelope of (F) is an envelope derived from Sendai virus.
(20) A kit for gene transfer, the kit comprising:
   (A) at least one lipid selected from the group consisting of sorbitan sesquioleate, sorbitan monolaurate, sorbitan monopalmitate, sorbitan monostearate, sorbitan trioleate, sorbitan monooleate, L-α-phosphatidylinositol, L-α-dioleoyl phosphatidylethanolamine, octadecylamine, hexadecylamine, DOTAP, and cardiolipin;
   (B) at least one protein selected from the group consisting of albumin, casein, gelatin, and sericin; and
   (C) at least one positively charged substance selected from the group consisting of protamine sulfate, polyarginine, polylysine, polyethyleneimine, and hexadimethrine bromide.
(21) The kit according to (20), further comprising (D) at least one extracellular matrix component selected from the group consisting of laminin, fibronectin, vitronectin, hyaluronic acid, entactin, elastin, tenascin, collagen, chondroitin sulfate, fibrin, and fibrinogen.
(22) The kit according to (20) or (21), further comprising (E) at least one member selected from the group consisting of chloroquine, quinacrine, hydroxychloroquine, cyclosporin, cyclophosphamide, tacrolimus, ascomycin, rapamycin, 2-cyano-3-(3,4-dihydroxyphenyl)-N-benzylacrylamide, amlexanox, N-[3-[[5-iodo-4-[[3-[(2-thienylcarbonyl)amino]propyl]amino]-2-pyrimidinyl]amino]phenyl]-1-pyrrolidinecarboxamide, doxorubicin, actinomycin D, aurintricarboxylic acid, paclitaxel, etoposide, N-[1-(3-(5-chloro-3-methylbenzo[b]thiophen-2-yl)-1-methyl-1H-pyrazol-5-yl)]-2-chlorobenzenesulfonamide, α-amanitin, β-amanitin, amlodipine, nifedipine, nicardipine, verapamil, diltiazem, trichostatin A, tubastatin A, suberoyl bis-hydroxamic acid, suberoylanilide hydroxamic acid, valproic acid, and phorbol 12-myristate 13-acetate.
(23) The kit according to any one of (20) to (22), further comprising (F) a viral envelope.
(24) The kit according to any one of (20) to (23), wherein the lipid of (A) is at least one member selected from the group consisting of sorbitan sesquioleate, sorbitan monolaurate, and hexadecylamine.
(25) The kit according to any one of (20) to (23), wherein the lipid of (A) is sorbitan sesquioleate.
(26) The kit according to any one of (20) to (25), wherein the positively charged substance of (C) is protamine sulfate.
(27) The kit according to any one of (21) to (26), wherein the extracellular matrix component of (D) is at least one member selected from the group consisting of laminin, vitronectin, and fibrinogen.
(28) The kit according to any one of (23) to (27), wherein the viral envelope of (F) is an envelope derived from Sendai virus.
(29) A composition for gene transfer, the composition comprising:
   (A) at least one lipid selected from the group consisting of sorbitan sesquioleate, sorbitan monolaurate, sorbitan monopalmitate, sorbitan monostearate, sorbitan trioleate, sorbitan monooleate, L-α-phosphatidylinositol, L-α-dioleoyl phosphatidylethanolamine, octadecylamine, hexadecylamine, DOTAP, and cardiolipin;
   (B) at least one protein selected from the group consisting of albumin and sericin; and
   (C) at least one positively charged substance selected from the group consisting of protamine sulfate, polyarginine, polylysine, and hexadimethrine bromide.
(30) The composition according to (29), further comprising (D) at least one extracellular matrix component selected from the group consisting of laminin, fibronectin, vitronectin, collagen, and fibrinogen.
(31) The composition according to (29) or (30), further comprising (E) at least one inhibitor for a Toll-like receptor, the inhibitor being selected from the group consisting of chloroquine and quinacrine.
(32) The composition according to any one of (29) to (31), further comprising (F) a viral envelope.
(33) The composition according to (29), wherein the lipid of (A) is at least one member selected from the group consisting of sorbitan sesquioleate, sorbitan monolaurate, and hexadecylamine.
(34) The composition according to (29), wherein the lipid of (A) is sorbitan sesquioleate.
(35) The composition according to (29), wherein the positively charged substance of (C) is protamine sulfate.
(36) The composition according to (30), wherein the extracellular matrix component of (D) is at least one member selected from the group consisting of laminin and fibrinogen.
(37) The composition according to (32), wherein the viral envelope of (F) is an envelope derived from Sendai virus.
(38) A composition for use in introducing a gene into a cell of a non-human animal in *in vivo* therapy, the composition comprising the composition according to (29).
(39) A method for introducing a gene into a cell in vitro, the method comprising bringing the composition according to (29) into contact with the cell, wherein the lipid, the protein, the positively charged substance, and the gene are all brought into contact with the cell simultaneously.

Moreover, the present inventor found that the effect of improving gene transfer efficiency due to component E is attained not only in the method of the present invention, but also in known gene transfer methods. More specifically, also disclosed herein are a method for improving gene transfer efficiency, the method using component E, and an agent for promoting gene transfer, the agent comprising component E as an active ingredient.

Particularly preferred as component E used in the method are quinacrine and N-[3-[[5-iodo-4-[[3-[(2-thienylcarbonyl)amino]propyl]amino]-2-pyrimidinyl]amino]phenyl]-1-pyrrolidinecarboxamide.

There is no particular limitation on known gene transfer methods. Examples of known gene transfer methods include methods using viral vectors, electroporation methods, ultrasound-mediated gene transfer methods, and methods in which plasmid DNA or the like is incorporated in carriers such as liposomes, polyethyleneimine, envelopes derived from viruses. Examples of known methods using viral vectors, electroporation methods, and ultrasound-mediated gene transfer methods include those disclosed in Kanarazu umaku iku idenshi dounyu to hatsugen kaiseki purotokoru [Protocols that work well for gene transfer and expression analysis], 2003, Yodosha. Examples of methods using liposomes or polyethyleneimine include those disclosed in Nonviral gene delivery:principle, limitations, and recent progress, The AAPS Journal, Dec. 2009; 11(4): 671-681. Examples of methods using envelopes derived from viruses include those disclosed in HVJ-envelope vector for gene transfer into central nervous system, Biochem Biophys Res Commun, Jan. 10, 2003; 300(2): 464-471. Known gene transfer methods may be those using commercially available products. Examples of commercially available products include Lipofectamine(trademark) LTX&PLUS reagent (Invitrogen), Lipofectamine(trademark) 3000 Reagent (Invitrogen), FuGENE(trademark) HD Transfection Reagent (Roche), jetPEI(trademark) (Polyplus), Polyethylenimine Max (Polysciences), Xfect(trademark) Transfection Reagent (Takara), Attractene Transfection Reagent (QIAGEN), Effectene(trademark) Transfection Reagent (QIAGEN), ViaFect(trademark) Transfection Reagent (Promega), X-tremeGENE 9 DNA Transfection Reagent (Roche), X-tremeGENE(trademark) HP DNA Transfection Reagent (Roche).

Disclosed herein are examples of a method for improving gene transfer efficiency.
(I) A method for improving gene transfer efficiency, the method comprising using (E) at least one member selected from the group consisting of chloroquine, quinacrine, hydroxychloroquine, cyclosporin, cyclophosphamide, tacrolimus, ascomycin, rapamycin, 2-cyano-3-(3,4-dihydroxyphenyl)-N-benzylacrylamide, amlexanox, N-[3-[[5-iodo-4-[[3-[(2-thienylcarbonyl)amino]propyl]amino]-2-pyrimidinyl]amino]phenyl]-1-pyrrolidinecarboxamide, doxorubicin, actinomycin D, aurintricarboxylic acid, paclitaxel, etoposide, N-[1-(3-(5-chloro-3-methylbenzo[b]thiophen-2-yl)-1-methyl-1H-pyrazol-5-yl)]-2-chlorobenzenesulfonamide, α-amanitin, β-amanitin, amlodipine, nifedipine, nicardipine, verapamil, diltiazem, trichostatin A, tubastatin A, suberoyl bis-hydroxamic acid, suberoylanilide hydroxamic acid, valproic acid, and phorbol 12-myristate 13-acetate.
(II) The method according to (I), wherein (E) is at least one member selected from the group consisting of quinacrine and N-[3-[[5-iodo-4-[[3-[(2-thienylcarbonyl)amino]propyl]amino]-2-pyrimidinyl]amino]phenyl]-1-pyrrolidinecarboxamide.
(III) The method according to (I) or (II), which is used in a gene transfer method using a liposome.
(IV) An agent for promoting gene transfer, the agent comprising, as an active ingredient, (E) at least one member selected from the group consisting of chloroquine, quinacrine, hydroxychloroquine, cyclosporin, cyclophosphamide, tacrolimus, ascomycin, rapamycin, 2-cyano-3-(3,4-dihydroxyphenyl)-N-benzylacrylamide, amlexanox, N-[3-[[5-iodo-4-[[3-[(2-thienylcarbonyl)amino]propyl]amino]-2-pyrimidinyl]amino]phenyl]-1-pyrrolidinecarboxamide, doxorubicin, actinomycin D, aurintricarboxylic acid, paclitaxel, etoposide, N-[1-(3-(5-chloro-3-methylbenzo[b]thiophen-2-yl)-1-methyl-1H-pyrazol-5-yl)]-2-chlorobenzenesulfonamide, α-amanitin, β-amanitin, amlodipine, nifedipine, nicardipine, verapamil, diltiazem, trichostatin A, tubastatin A, suberoyl bis-hydroxamic acid, suberoylanilide hydroxamic acid, valproic acid, and phorbol 12-myristate 13-acetate.
(V) The agent for promoting gene transfer according to (IV), wherein (E) is at least one member selected from the group consisting of quinacrine and N-[3-[[5-iodo-4-[[3-[(2-thienylcarbonyl)amino]propyl]amino]-2-pyrimidinyl]amino]phenyl]-1-pyrrolidinecarboxamide.

### Examples

Examples are given below to illustrate the present invention in more detail. In the following description, "%" means W/V% unless otherwise specified.

### Example 1

A549 cells, which are adherent cultured cells, were seeded in a 96-well plate at a cell density of 4 × 10³ cells/well one day before transfection. THP-1 cells, which are suspension cultured cells, were seeded in a 96-well plate at a cell density of 2 × 10⁴ cells/well on the day of transfection.

42 µL of 10 mg/mL sericin (Wako Pure Chemical Industries, Ltd., trade name: Pure Sericin) PBS solution was added to 3 µL of a 1/2% sorbitan sesquioleate (Nacalai Tesque, Inc.) ethanol solution, and the mixture was mixed by pipetting.

A sample not containing sorbitan sesquioleate was prepared by adding 42 µL of a 10 mg/mL sericin PBS solution to 3 µL of ethanol and mixing the mixture by pipetting.

A sample not containing sericin was prepared by adding 42 µL of PBS to 3 µL of a 1/2% sorbitan sesquioleate ethanol solution and mixing the mixture by pipetting.

A sample not containing sorbitan sesquioleate or sericin was prepared by adding 42 µL of PBS to 3 µL of ethanol and mixing the mixture by pipetting.

15 µL of each of the above prepared solutions was individually added to 1.25 µL of a 1 mg/mL pTurboGFP-N plasmid solution (Evrogen), and each mixture was mixed by pipetting. 6.3 µL of a 1 mg/mL protamine sulfate PBS solution was then added to each mixture, thereby preparing test compositions. Each of these test compositions was added at 5 µL/well to the cell culture media.

The next day, whether gene transfer was achieved was confirmed by observing green fluorescent protein (TurboGFP, Evrogen) produced from the pTurboGFP-N plasmid with a fluorescence microscope. Fig. 3 shows the results.

Almost no gene transfer was observed when only component C was used, when the mixture of component B and component C was used, and when the mixture of component A and component C was used. In contrast, the results revealed that when the composition of the present invention, which comprises component A, component B, and component C, was used, the gene was introduced into the A549 cells and the THP-1 cells.

### Example 2

Jurkat cells, which are suspension cultured cells, were seeded in a 96-well plate at a cell density of 4 × 10⁴ cells/well on the day of transfection.

280 µL of a 15 mg/mL sericin (Wako Pure Chemical Industries, Ltd., trade name: Pure Sericin) PBS solution was added to 20 µL of a 1/2% sorbitan sesquioleate (Nacalai Tesque, Inc.) ethanol solution, and the mixture was mixed by pipetting.

As a virus-derived envelope, a 47.8 HAU/µL inactivated HVJ envelope (GenomONE-CF: registered trademark, produced by Ishihara Sangyo Kaisha, Ltd.) suspension, which is derived from Sendai virus, was used in the experiment.

The inactivated HVJ envelope suspension was diluted 2-fold, 4-fold, 8-fold, 16-fold, and 32-fold with PBS. 8 µL of a 0.3% Triton X-100 PBS solution was added to 4 µL of each of these diluted, inactivated HVJ envelope suspensions, and each mixture was centrifuged at 4°C, 10,000 g for 5 minutes with a high-speed refrigerated microcentrifuge, thereby pelleting down the inactivated HVJ envelope. After the supernatant from each mixture was removed, the inactivated HVJ envelope in which the permeability of the envelope membrane was improved by the Triton X-100 treatment was suspended in 12.5 µL of a 1/10 mg/mL pCMV-GL3 plasmid solution. 4 µL of PBS was added to 12.5 µL of each of the individual HVJ envelope suspensions containing the plasmid or to a 1/10 mg/mL pCMV-GL3 plasmid solution, and 20 µL of the liquid mixture of sorbitan sesquioleate and sericin prepared in advance was further added to each mixture. After each of the resulting mixtures was mixed by pipetting, 6.25 µL of a 1 mg/mL protamine sulfate PBS solution was added to each mixture, thereby preparing test compositions. Each of these test compositions was added at 9.5 µL/well to the cell culture medium.

The next day, whether gene transfer was achieved was confirmed by measuring the enzyme activity (relative value) of luciferase protein produced from the pCMV-GL3 plasmid by using ONE-Glo Luciferase Assay System (Promega). Fig. 4 shows the results.

The results revealed that use of component F improves gene transfer efficiency of the composition of the present invention.

### Example 3

NIH-3T3 cells, which are adherent cultured cells, were seeded in a 96-well plate at a cell density of 4 × 10³ cells/well one day before transfection.

84 µL of a 10 mg/mL sericin (Wako Pure Chemical Industries, Ltd., trade name: Pure Sericin) aqueous solution was added to 6 µL of a 1/2% sorbitan sesquioleate (Nacalai Tesque, Inc.) ethanol solution, and the mixture was mixed by pipetting.

21 µL of a 1/40 mg/mL laminin (SIGMA) aqueous solution or water was individually added to 1.875 µL of a 1 mg/mL pCMV-GL3 plasmid solution, and each mixture was mixed by pipetting. After 7.6 µL of each of these liquid mixtures was transferred to separate tubes, 7.5 µL of the liquid mixture of sorbitan sesquioleate and sericin prepared in advance was added thereto. Each mixture was mixed by pipetting, and 3.1 µL of a 1 mg/mL protamine sulfate PBS solution was then added to each of the resulting mixtures, thereby preparing test compositions. Each of these test compositions was added at 8.1 µL/well to the cell culture medium.

The next day, whether gene transfer was achieved was confirmed by measuring the enzyme activity (relative value) of luciferase protein produced from the pCMV-GL3 plasmid by using ONE-Glo Luciferase Assay System (Promega). Fig. 5 shows the results.

### Example 4

NIH-3T3 cells, which are adherent cultured cells, were seeded in a 96-well plate at a cell density of 4 × 10³ cells/well one day before transfection.

210 µL of a 15 mg/mL sericin PBS solution was added to 15 µL of a 1/2% sorbitan sesquioleate ethanol solution, and the mixture was mixed by pipetting.

As a virus-derived envelope, a 47.8 HAU/µL inactivated HVJ envelope (GenomONE-CF: registered trademark, produced by Ishihara Sangyo Kaisha, Ltd.) suspension, which is derived from Sendai virus, was used in the experiment.

54 µL of a PBS solution and 6 µL of a 2% Triton X-100 PBS solution were added to 6 µL of the inactivated HVJ envelope suspension, and the mixture was centrifuged at 4°C, 10,000 g for 5 minutes with a high-speed refrigerated microcentrifuge, thereby pelletting down the inactivated HVJ envelope. After the supernatant was removed, the inactivated HVJ envelope in which the permeability of the envelope membrane was improved by the Triton X-100 treatment was suspended in 15 µL of a 1 mg/mL pCMV-GL3 plasmid solution. After 1.25 µL of the liquid mixture of the inactivated HVJ envelope and the pCMV-GL3 plasmid was transferred to each tube, 4 µL of a 1 mM chloroquine (SIGMA) PBS solution, a 1/3 mM quinacrine (Wako Pure Chemical Industries, Ltd.) PBS solution, or PBS was individually added thereto, and 15 µL of the liquid mixture of sorbitan sesquioleate and sericin prepared in advance was further added. Each mixture was mixed by pipetting, and 6.3 µL of a 1 mg/mL protamine sulfate PBS solution was then added to each of the resulting mixtures, thereby preparing test compositions. Each of these test compositions was added at 6 µL/well to the cell culture medium.

The next day, whether gene transfer was achieved was confirmed by measuring the enzyme activity (relative value) of luciferase protein produced from the pCMV-GL3 plasmid by using ONE-Glo Luciferase Assay System (Promega). Fig. 6 shows the results.

### Example 5

A549 cells and RAW264.7 cells, both of which are adherent cultured cells, were respectively seeded in a 96-well plate at a cell density of 4 × 10³ cells/well and at a cell density of 2 × 10⁴ cells/well one day before transfection.

28 µL of a 15 mg/mL sericin (Wako Pure Chemical Industries, Ltd., trade name: Pure Sericin) PBS solution, 1, 1/3, or 1/9 mg/mL albumin (SIGMA) aqueous solution, or water was individually added to 2 µL of a 1/2% sorbitan sesquioleate (Nacalai Tesque, Inc.) ethanol solution, and each mixture was mixed by pipetting.

As a virus-derived envelope, a 47.8 HAU/µL inactivated HVJ envelope (GenomONE-CF: registered trademark, produced by Ishihara Sangyo Kaisha, Ltd.) suspension, which is derived from Sendai virus, was used in the experiment.

4 µL of a 0.2% Triton X-100 PBS solution was added to 4 µL of the inactivated HVJ envelope suspension, and the mixture was centrifuged at 4°C, 10,000 g for 5 minutes with a high-speed refrigerated microcentrifuge, thereby pelletting down the inactivated HVJ envelope. After the supernatant was removed, the inactivated HVJ envelope in which the permeability of the envelope membrane was improved by the Triton X-100 treatment was suspended in 100 µL of a 1/10 mg/mL pCMV-GL3 plasmid solution. After 12.5 µL of the liquid mixture of the inactivated HVJ envelope and the pCMV-GL3 plasmid was transferred to each tube, 15 µL of the liquid mixture of sorbitan sesquioleate and sericin prepared in advance or the liquid mixture of sorbitan sesquioleate and albumin prepared in advance was individually added thereto. Each mixture was mixed by pipetting, and 6.25 µL of a 1 mg/mL protamine sulfate PBS solution was then added to each of the resulting mixtures, thereby preparing test compositions. Each of these test compositions was added at 7.5 µL/well to the cell culture media.

The next day, whether gene transfer was achieved was confirmed by measuring the enzyme activity (relative value) of luciferase protein produced from the pCMV-GL3 plasmid by using ONE-Glo Luciferase Assay System (Promega). Fig. 7 shows the results.

### Example 6

K562 cells, which are suspension cultured cells, were seeded on a 96-well plate at a cell density of 1 × 10⁴ cells/well on the day of transfection.

3 µL of a 1/20% hexadecylamine (Wako Pure Chemical Industries, Ltd.) ethanol solution was added to 3 µL of a 1/2% sorbitan sesquioleate (Nacalai Tesque, Inc.) ethanol solution, and 42 µL of a 10 mg/mL sericin aqueous solution was further added thereto, and the mixture was mixed by pipetting.

A sample not containing hexadecylamine was prepared by adding 3 µL of ethanol to 3 µL of a 1/2% sorbitan sesquioleate ethanol solution, further adding 42 µL of a 10 mg/mL sericin aqueous solution thereto, and mixing the mixture by pipetting.

19.2 µL of each of the above prepared solutions was individually added to 1.5 µL of a 1 mg/mL pCMV-GL3 plasmid solution, and each mixture was mixed by pipetting. 7.5 µL of a 1 mg/mL protamine sulfate PBS solution was then added to each of the resulting mixtures, thereby preparing test compositions. Each of these test compositions was added at 5 µL/well to the cell culture medium.

The next day, whether gene transfer was achieved was confirmed by measuring the enzyme activity (relative value) of luciferase protein produced from the pCMV-GL3 plasmid by using ONE-Glo Luciferase Assay System (Promega). Fig. 8 shows the results.

### Example 7

Jurkat cells, which are suspension cultured cells, were seeded in a 96-well plate at a cell density of 4 × 10⁴ cells/well on the day of transfection.

112 µL of a 15 mg/mL sericin (Wako Pure Chemical Industries, Ltd., trade name: Pure Sericin) PBS solution was added to 8 µL of a 1/2% sorbitan sesquioleate (Nacalai Tesque, Inc.) ethanol solution, and the mixture was mixed by pipetting.

As a virus-derived envelope, a 47.8 HAU/µL inactivated HVJ envelope (GenomONE-CF: registered trademark, produced by Ishihara Sangyo Kaisha, Ltd.) suspension, which is derived from Sendai virus, was used in the experiment.

10 µL of a 0.2% Triton X-100 PBS solution was added to 1 µL of the inactivated HVJ envelope suspension, and the mixture was centrifuged at 4°C, 10,000 g for 5 minutes with a high-speed refrigerated microcentrifuge, thereby pelletting down the inactivated HVJ envelope. After the supernatant was removed, the inactivated HVJ envelope in which the permeability of the envelope membrane was improved by the Triton X-100 treatment was suspended in 25 µL of a 1/10 mg/mL pCMV-GL3 plasmid solution.

30 µL of the liquid mixture of sorbitan sesquioleate and sericin prepared in advance was added to the HVJ envelope containing pCMV-GL3 plasmid or a pCMV-GL3 plasmid solution, and each mixture was mixed by pipetting. 12.5 µL of a 1 mg/mL protamine sulfate PBS solution was then added to each of the resulting mixtures, thereby preparing test compositions. Each of these test compositions was added at 7.5 µL/well to the cell culture medium.

For comparison, gene transfer using Lipofectamine LTX&PLUS reagent (Invitrogen), which is a commercially available gene transfer reagent, was performed as follows. 0.1 µL of a 1 mg/mL pCMV-GL3 plasmid solution and 0.1 µL of PLUS reagent were added to 20 µL of Opti-MEM I Reduced Serum Medium (Gibco), and the mixture was allowed to stand for 5 minutes. 0.55 µL of Lipofectamine LTX reagent was further added thereto, and the mixture was allowed to stand for 25 minutes, after which it was added at 20 µL/well to the cell culture medium.

The next day, whether gene transfer was achieved was confirmed by measuring the enzyme activity (relative value) of luciferase protein produced from the pCMV-GL3 plasmid by using ONE-Glo Luciferase Assay System (Promega). Fig. 9 shows the results.

The results revealed that the composition of the present invention has notably high gene transfer efficiency compared with Lipofectamine LTX&PLUS reagent (Invitrogen), which is a commercially available gene transfer reagent.

### Example 8

A549 cells, which are adherent cultured cells, were seeded in a 96-well plate at a cell density of 4 × 10³ cells/well one day before transfection.

350 µL of a 15 mg/mL sericin (Wako Pure Chemical Industries, Ltd., trade name: Pure Sericin) PBS solution was added to 25 µL of a 1/2% sorbitan sesquioleate (Nacalai Tesque, Inc.) ethanol solution, and the mixture was mixed by pipetting.

9 µL of 50/3 µM compound A (InvivoGen) or water was individually added to 30 µL of a 1/10 mg/mL pCMV-GL3 plasmid solution, and 36 µL of the liquid mixture of sorbitan sesquioleate and sericin prepared in advance was further added to each mixture. After each mixture was mixed by pipetting, 15 µL of a 1 mg/mL protamine sulfate PBS solution was added to each of the resulting mixtures, thereby preparing test compositions. Each of these test compositions was added at 20 µL/well to the cell culture medium.

The next day, whether gene transfer was achieved was confirmed by measuring the enzyme activity (relative value) of luciferase protein produced from the pCMV-GL3 plasmid by using ONE-Glo Luciferase Assay System (Promega). Fig. 10 shows the results.

### Example 9

RAW264.7 cells, which are adherent cultured cells, were seeded in a 96-well plate at a cell density of 2 × 10⁴ cells/well one day before transfection.

Jurkat cells, U937 cells, K562 cells, and THP-1 cells, all of which are suspension cultured cells, were respectively seeded at a cell density of 4 × 10⁴ cells/well, at a cell density of 4 × 10⁴ cells/well, at a cell density of 2 × 10⁴ cells/well, and at a cell density of 8 × 10⁴ cells/well on the day of transfection.

84 µL of a 15 mg/mL sericin (Wako Pure Chemical Industries, Ltd., trade name: Pure Sericin) PBS solution was added to 6 µL of a 1/2% sorbitan sesquioleate (Nacalai Tesque, Inc.) ethanol solution, and the mixture was mixed by pipetting.

A sample not containing sorbitan sesquioleate was prepared by adding 84 µL of a 15 mg/mL sericin PBS solution to 6 µL of ethanol and mixing the mixture by pipetting.

A sample not containing sericin was prepared by adding 84 µL of PBS to 6 µL of a 1/2% sorbitan sesquioleate ethanol solution and mixing the mixture by pipetting.

36 µL of each of the above prepared solutions was individually added to 30 µL of a 1/10 mg/mL pCMV-GL3 plasmid solution, and each mixture was mixed by pipetting. 15 µL of a 1 mg/mL protamine sulfate PBS solution was then added to each of the resulting mixtures, thereby preparing test compositions. Each of these test compositions was added at 15 µL/well to the cell culture media.

The next day, whether gene transfer was achieved was confirmed by measuring the enzyme activity (relative value) of luciferase protein produced from the pCMV-GL3 plasmid by using ONE-Glo Luciferase Assay System (Promega). Table 1 shows the results.

**Table 1**

| Kind of cells | Luciferase gene expression level (relative value) | | |
|---|---|---|---|
| | Sorbitan sesquioleate | | Sorbitan sesquioleate |
| | | Sericin | Sericin |
| | Protamine sulfate | Protamine sulfate | Protamine sulfate |
| RAW264.7 | 1,747 | 28 | 47,028 |
| Jurkat | 1,643 | 9 | 70,172 |
| K562 | 440 | 9 | 394,125 |
| THP-1 | 1,996 | 18 | 9,680 |
| U937 | 12 | 8 | 1,403 |

As shown in Table 1, the results revealed that almost no gene transfer was observed when the mixture of component A and component C was used and when the mixture of component B and component C was used, whereas use of the composition of the present invention, which comprises component A, component B, and component C, achieved gene transfer in both the adherent cells and suspension cells with high efficiency.

### Example 10

Jurkat cells, U937 cells, K562 cells, and THP-1 cells, all of which are suspension cultured cells, were respectively seeded at a cell density of 4 × 10⁴ cells/well, at a cell density of 4 × 10⁴ cells/well, at a cell density of 2 × 10⁴ cells/well, and at a cell density of 8 × 10⁴ cells/well on the day of transfection.

84 µL of a 15 mg/mL sericin (Wako Pure Chemical Industries, Ltd., trade name: Pure Sericin) PBS solution was added to 6 µL of a 1/2% sorbitan sesquioleate (Nacalai Tesque, Inc.) ethanol solution, and the mixture was mixed by pipetting.

A sample not containing sericin was prepared by adding 84 µL of PBS to 6 µL of a 1/2% sorbitan sesquioleate ethanol solution and mixing the mixture by pipetting.

30 µL of each of the above prepared solutions was individually added to 25 µL of a 1/10 mg/mL pCMV-GL3 plasmid solution, and each mixture was mixed by pipetting. 12.5 µL of a 1 mg/mL protamine sulfate PBS solution was then added to each of the resulting mixtures, thereby preparing test compositions. Each of these test compositions was added at 15 µL/well to the cell culture media. Immediately after transfection, PBS was added at 4.7 µL/well to the sample containing sericin, and a 15 mg/mL sericin PBS solution was added at 4.7 µL/well to the sample not containing sericin.

The next day, whether gene transfer was achieved was confirmed by measuring the enzyme activity (relative value) of luciferase protein produced from the pCMV-GL3 plasmid by using ONE-Glo Luciferase Assay System (Promega). Table 2 shows the results.

**Table 2**

| Kind of cells | Luciferase gene expression level (relative value) | |
|---|---|---|
| | Sorbitan sesquioleate | Sorbitan sesquioleate |
| | | Sericin |
| | Protamine sulfate | Protamine sulfate |
| | Addition of sericin to cell culture media immediately after transfection | Addition of PBS to cell culture media immediately after transfection |
| Jurkat | 11,634 | 74,779 |
| K562 | 6,508 | 699,166 |
| THP-1 | 1,351 | 7,191 |
| U937 | 20 | 1,195 |

As shown in Table 2, the results revealed that the use of the mixture of component A, component B, and component C achieved gene transfer with higher efficiency.

### Example 11

A549 cells and NIH-3T3 cells, both of which are adherent cultured cells, were individually seeded in a 96-well plate at a cell density of 4 × 10³ cells/well one day before transfection.

84 µL of a 10 mg/mL sericin (Wako Pure Chemical Industries, Ltd., trade name: Pure Sericin) aqueous solution was added to 6 µL of a 1/2% sorbitan sesquioleate (Nacalai Tesque, Inc.) ethanol solution, and the mixture was mixed by pipetting.

As a virus-derived envelope, a 47.8 HAU/µL inactivated HVJ envelope (GenomONE-CF: registered trademark, produced by Ishihara Sangyo Kaisha, Ltd.) suspension, which is derived from Sendai virus, was used in the experiment.

After 54 µL of PBS was added to 6 µL of the inactivated HVJ envelope suspension, 6 µL of a 2% Triton X-100 PBS solution was added thereto. The mixture was centrifuged at 4°C, 10,000 g for 5 minutes with a high-speed refrigerated microcentrifuge, thereby pelletting down the inactivated HVJ envelope. After the supernatant was removed, the inactivated HVJ envelope in which the permeability of the envelope membrane was improved by the Triton X-100 treatment was suspended in 15 µL of a 1 mg/mL pCMV-GL3 plasmid solution. After 2.5 µL of the liquid mixture of the inactivated HVJ envelope and the pCMV-GL3 plasmid was transferred to each tube, 28 µL of a 1/20 mg/mL laminin (SIGMA) aqueous solution or water was individually added thereto, and each mixture was mixed by pipetting. 7.6 µL of each of these liquid mixtures was transferred to separate tubes, and 7.5 µL of the liquid mixture of sorbitan sesquioleate and sericin prepared in advance, 7 µL of a 10 mg/mL sericin aqueous solution and 0.5 µL of water, 0.5 µL of a 1/2% sorbitan sesquioleate ethanol solution and 7 µL of water, or 7.5 µL of water was individually added to each mixture. After each mixture was mixed by pipetting, 3.1 µL of a 1 mg/mL protamine sulfate PBS solution was added to each of the resulting mixtures, thereby preparing test compositions. Each of these test compositions was added at 8.1 µL/well to the cell culture media.

The next day, whether gene transfer was achieved was confirmed by measuring the enzyme activity (relative value) of luciferase protein produced from the pCMV-GL3 plasmid by using ONE-Glo Luciferase Assay System (Promega).

**Table 3**

| Kind of cells | Luciferase gene expression level (relative value) | | | | | |
|---|---|---|---|---|---|---|
| | | | | Sorbitan sesquioleate | Sorbitan sesquioleate | Sorbitan sesquioleate |
| | | Sericin | | | Sericin | Sericin |
| | Protamine sulfate | Protamine sulfate | Protamine sulfate | Protamine sulfate | Protamine sulfate | Protamine sulfate |
| | | | Laminin | | | Laminin |
| | HVJ envelope | HVJ envelope | HVJ envelope | HVJ envelope | HVJ envelope | HVJ envelope |
| A549 | 521 | 3,270 | 23,904 | 1,284 | 139,393 | 301,851 |
| NIH-3T3 | 27,347 | 28,026 | 356,656 | 27,030 | 394,534 | 1,592,775 |

As shown in Table 3, the results revealed the following: even in only component C, the mixture of component B and component C, the mixture of component C and component D, and the mixture of component A and component C, gene transfer was observed when component F was used in combination; when the composition of the present invention, i.e., a mixture of component A, component B, and component C or a mixture of component A, component B, component C, and component E, was used in combination with component F, gene transfer was achieved with higher efficiency.

### Example 12

NIH-3T3 cells, which are adherent cultured cells, were seeded in a 96-well plate at a cell density of 4 × 10³ cells/well one day before transfection. Jurkat cells, which are suspension cultured cells, were seeded in a 96-well plate at a cell density of 4 × 10⁴ cells/well on the day of transfection.

16.8 µL of a 5 mg/mL sericin PBS solution was added to 1.2 µL of a 1/4% sorbitan sesquioleate (Nacalai Tesque, Inc.) ethanol solution, and the mixture was mixed by pipetting.

16.8 µL of a 5 mg/mL sericin PBS solution was added to 1.2 µL of a 1/4% sorbitan monooleate (SIGMA) ethanol solution, and the mixture was mixed by pipetting.

0.6 µL of a 1/5% L-α-dioleoyl phosphatidylethanolamine (Wako Pure Chemical Industries, Ltd.; hereinafter referred to as "DOPE") ethanol solution or 0.6 µL of ethanol was individually added to 0.6 µL of a 1/5% cardiolipin (SIGMA) ethanol solution. 16.8 µL of a 5 mg/mL sericin PBS solution was further added to each mixture, and each of the resulting mixtures was mixed by pipetting.

15 µL of a 1/10 mg/mL pCMV-GL3 plasmid solution was added to 18 µL of each of the above prepared solutions, and each mixture was mixed by pipetting. 7.5 µL of a 1 mg/mL protamine sulfate PBS solution was then added to each of the resulting mixtures, thereby preparing test compositions. Each of these test compositions was added at 15 µL/well to the cell culture media.

The next day, whether gene transfer was achieved was confirmed by measuring the enzyme activity (relative value) of luciferase protein produced from the pCMV-GL3 plasmid by using ONE-Glo Luciferase Assay System (Promega).

**Table 4**

| Kind of cells | Luciferase gene expression level (relative value) | | | |
|---|---|---|---|---|
| | Cardiolipin | Cardiolipin | Sorbitan monooleate | Sorbitan sesquioleate |
| | | DOPE | | |
| | Sericin | Sericin | Sericin | Sericin |
| | Protamine sulfate | Protamine sulfate | Protamine sulfate | Protamine sulfate |
| NIH-3T3 | 270,930 | 624,517 | 1,398,496 | 2,615,005 |
| Jurkat | 3,212 | 7,115 | 68,076 | 96,959 |

As shown in Table 4, the results revealed that the composition of the present invention containing sorbitan sesquioleate, sorbitan monooleate, cardiolipin, or cardiolipin and DOPE as component A makes it possible to introduce a gene into NIH-3T3 cells and Jurkat cells.

### Example 13

NIH-3T3 cells, which are adherent cultured cells, were seeded in a 96-well plate at a cell density of 4 × 10³ cells/well one day before transfection.

11.2 µL of 1/4, 1/2, 3, 5, 20, 30, or 50 mg/mL sericin PBS solution or PBS was individually added to 0.8 µL of a 1/2% sorbitan sesquioleate (Nacalai Tesque, Inc.) ethanol solution, and each mixture was mixed by pipetting.

10 µL of a 1/10 mg/mL pCMV-GL3 plasmid solution was added to 12 µL of each of the prepared solutions above, and each mixture was mixed by pipetting. 5 µL of a 1 mg/mL protamine sulfate PBS solution was then added to each of the resulting mixtures, thereby preparing test compositions. Each of these test compositions was added at 15 µL/well to the cell culture medium.

The next day, whether gene transfer was achieved was confirmed by measuring the enzyme activity (relative value) of luciferase protein produced from the pCMV-GL3 plasmid by using ONE-Glo Luciferase Assay System (Promega).

**Table 5**

| Kind of cells | Luciferase gene expression level (relative value) | | | | | | | |
|---|---|---|---|---|---|---|---|---|
| | Sericin concentration | | | | | | | |
| | 0 mg/mL | 1/4 mg/mL | 1/2 mg/mL | 3 mg/mL | 5 mg/mL | 20 mg/mL | 30 mg/mL | 50 mg/mL |
| NIH-3T3 | 346 | 77,190 | 285,344 | 761,785 | 729,703 | 762,661 | 458,447 | 24,194 |

As shown in Table 5, gene expression was improved when 1/4 to 50 mg/mL sericin was used compared with the case where sericin was not used.

### Example 14

NIH-3T3 cells, which are adherent cultured cells, were seeded in a 96-well plate at a cell density of 4 × 10³ cells/well one day before transfection.

11.2 µL of a 10 mg/mL sericin (Wako Pure Chemical Industries, Ltd.; trade name: Pure Sericin) PBS solution was added to 0.8 µL of each of 1/50, 1/10, 1/2, and 5/2% sorbitan sesquioleate (Nacalai Tesque, Inc.) ethanol solutions, and each mixture was mixed by pipetting.

10 µL of a 1/10 mg/mL pCMV-GL3 plasmid solution was added to 12 µL of each of the prepared solutions above, and each mixture was mixed by pipetting. 5 µL of a 1 mg/mL protamine sulfate PBS solution was then added to each of the resulting mixtures, thereby preparing test compositions. Each of these test compositions was added at 15 µL/well to the cell culture medium.

The next day, whether gene transfer was achieved was confirmed by measuring the enzyme activity (relative value) of luciferase protein produced from the pCMV-GL3 plasmid by using ONE-Glo Luciferase Assay System (Promega).

**Table 6**

| Kind of cells | Luciferase gene expression level (relative value) | | | |
|---|---|---|---|---|
| | Sorbitan sesquioleate concentration | | | |
| | 1/50% | 1/10% | 1/2% | 5/2% |
| NIH-3T3 | 887 | 291,463 | 1,349,057 | 425,949 |

As shown in Table 6, the results revealed that gene expression was observed when 1/50 to 5/2% sorbitan sesquioleate was used.

### Example 15

Jurkat cells, which are suspension cultured cells, were seeded in a 96-well plate at a cell density of 4 × 10⁴ cells/well on the day of transfection.

Test compositions were prepared using pCMV-GL3, sorbitan sesquioleate, sericin, and protamine sulfate by changing the mixing order as shown in (a) to (d) below.
(a) 28 µL of a 15 mg/mL sericin (Wako Pure Chemical Industries, Ltd.; trade name: Pure Sericin) PBS solution was added to 2 µL of a 1/2% sorbitan sesquioleate (Nacalai Tesque, Inc.) ethanol solution, and the mixture was mixed by pipetting.
   30 µL of the prepared solution above was added to 25 µL of a 1/10 mg/mL pCMV-GL3 plasmid solution, and the mixture was mixed by pipetting. 12.5 µL of a 1 mg/mL protamine sulfate PBS solution was then added thereto, thereby preparing a test composition.
(b) 28 µL of a 15 mg/mL sericin (Wako Pure Chemical Industries, Ltd.; trade name: Pure Sericin) PBS solution was added to 2 µL of a 1/2% sorbitan sesquioleate (Nacalai Tesque, Inc.) ethanol solution, and the mixture was mixed by pipetting.
   12.5 µL of a 1 mg/mL protamine sulfate PBS solution was added to 25 µL of a 1/10 mg/mL pCMV-GL3 plasmid solution, and the mixture was mixed by pipetting. 30 µL of the prepared solution above was then added thereto, thereby preparing a test composition.
(c) 28 µL of a 15 mg/mL sericin (Wako Pure Chemical Industries, Ltd.; trade name: Pure Sericin) PBS solution was added to 25 µL of a 1/10 mg/mL pCMV-GL3 plasmid solution, and the mixture was mixed by pipetting.
   53 µL of the prepared solution above was added to 2 µL of a 1/2% sorbitan sesquioleate (Nacalai Tesque, Inc.) ethanol solution, and the mixture was mixed by pipetting. 12.5 µL of a 1 mg/mL protamine sulfate PBS solution was then added thereto, thereby preparing a test composition.
(d) 28 µL of a 15 mg/mL sericin PBS solution and 12.5 µL of a 1 mg/mL protamine sulfate PBS solution were added to 25 µL of a 1/10 mg/mL pCMV-GL3 plasmid solution, and the mixture was mixed by pipetting.

65.5 µL of the prepared solution above was added to 2 µL of a 1/2% sorbitan sesquioleate (Nacalai Tesque, Inc.) ethanol solution, and the mixture was mixed by pipetting, thereby preparing a test composition.

Each of the test compositions prepared by the procedures (a) to (d) was added at 15 µL/well to the cell culture medium.

The next day, whether gene transfer was achieved was confirmed by measuring the enzyme activity (relative value) of luciferase protein produced from the pCMV-GL3 plasmid by using ONE-Glo Luciferase Assay System (Promega).

**Table 7**

| Kind of cells | Luciferase gene expression level (relative value) | | | |
|---|---|---|---|---|
| | Mixing order | | | |
| | (a) | (b) | (c) | (d) |
| Jurkat | 52,642 | 40,083 | 44,294 | 39,765 |

As shown in Table 7, there was no large difference in gene expression even when the composition of the present invention was prepared in any mixing order. This result confirmed that the composition of the present invention can be prepared without limiting the order to that shown in Fig. 2.

### Example 16

A549 cells and RAW264.7 cells, both of which are adherent cultured cells, were respectively seeded in a 96-well plate at a cell density of 4 × 10³ cells/well and at a cell density of 2 × 10⁴ cells/well one day before transfection.

K562 cells, which are suspension cultured cells, were seeded in a 96-well plate at a cell density of 2 × 10⁴ cells/well on the day of transfection.

154 µL of a 15 mg/mL sericin PBS solution was added to 11 µL of a 1/2% sorbitan sesquioleate (Nacalai Tesque, Inc.) ethanol solution, and the mixture was mixed by pipetting.

48 µL of the prepared solution above was added to 40 µL of a 1/10 mg/mL pCMV-GL3 plasmid solution, and the mixture was mixed by pipetting. 20 µL of a 1 mg/mL protamine sulfate PBS solution was then added to prepare a test composition (prepared for each kind of cells; total of three tubes). The test composition was added at 15 µL/well to the cell culture media.

Gene transfer using Lipofectamine LTX&PLUS reagent (Invitrogen) was performed as follows. 0.7 µL of a 1 mg/mL pCMV-GL3 plasmid solution and 0.7 µL of a PLUS reagent were added to 140 µL of Opti-MEM I Reduced Serum Medium (Gibco), and the mixture was allowed to stand for 5 minutes. 2.8 µL of Lipofectamine LTX reagent was further added thereto, and the mixture was allowed to stand for 25 minutes, after which it was added at 20 µL/well to the cell culture media.

Immediately before these gene transfer vectors were added to the cell culture media, 20 µM compound A DMSO aqueous solution or water was added at 5 µL/well to the cell culture media.

The next day, whether gene transfer was achieved was confirmed by measuring the enzyme activity (relative value) of luciferase protein produced from the pCMV-GL3 plasmid by using ONE-Glo Luciferase Assay System (Promega).

**Table 8**

| Kind of cells | Luciferase gene expression level (relative value) | | | |
|---|---|---|---|---|
| | Sorbitan sesquioleate | Sorbitan sesquioleate | LipofectAmine LTX | LipofectAmine LTX |
| | Sericin | Sericin | | |
| | Protamine sulfate | Protamine sulfate | | |
| | | Compound A | | Compound A |
| A549 | 663,220 | 928,553 | 291,872 | 659,846 |
| RAW264.7 | 5,745 | 101,071 | 5,377 | 86,598 |
| K562 | 415,210 | 1,270,938 | 33,178 | 1,527,805 |

As shown in Table 8, when compound A was used in combination with the composition of the present invention or Lipofectamine LTX&PLUS reagent, which is a commercially available gene transfer reagent, improvement in the gene expression was confirmed in both cases.

### Example 17

RAW264.7 cells, which are adherent cultured cells, were seeded in a 96-well plate at a cell density of 2 × 10⁴ cells/well one day before transfection.

33.6 µL of a 15 mg/mL sericin PBS solution was added to 2.4 µL of a 1/2% sorbitan sesquioleate (Nacalai Tesque, Inc.) ethanol solution, and the mixture was mixed by pipetting. 30 µL of a 1/10 mg/mL pCMV-GL3 plasmid solution was added to the prepared solution above, and the mixture was mixed by pipetting. 15 µL of 1 mg/mL protamine sulfate PBS solution was then added thereto, thereby preparing a test composition. This test composition was added at 15 µL/well to the cell culture medium.

Gene transfer using FuGENE HD transfection reagent (Roche) was performed as follows. 1 µL of a 1 mg/mL pCMV-GL3 plasmid solution was added to 50 µL of Opti-MEM I Reduced Serum Medium (Gibco), and the mixture was mixed by pipetting. 0.5 µL of FuGENE HD transfection reagent was added to 12.5 µL of this prepared solution, and the mixture was allowed to stand for 15 minutes, after which it was added at 5.2 µL/well to the cell culture medium.

Gene transfer using Lipofectamine LTX&PLUS reagent (Invitrogen) was performed as follows. 0.2 µL of a 1 mg/mL pCMV-GL3 plasmid solution and 0.2 µL of PLUS reagent were added to 40 µL of Opti-MEM I Reduced Serum Medium (Gibco), and the mixture was allowed to stand fro 5 minutes. 0.8 µL of Lipofectamine LTX reagent was further added thereto, and the mixture was allowed to stand for 25 minutes, after which it was added at 20 µL/well to the cell culture medium.

Gene transfer using Polyethylenimine Max (Polyscience) was performed as follows. 13.5 µL of water was added to 1.5 µL of 1 mg/mL Polyethylenimine Max (pH of 7, MW of 25,000). 15 µL of a 1/30 mg/mL pCMV-GL3 plasmid solution was added thereto, and the mixture was allowed to stand for 20 minutes, after which it was added at 5 µL/well to the cell culture medium.

Immediately before these gene transfer vectors were added to the cell culture medium, a 20 µM DMSO aqueous solution of compound A or water was added at 5 µL/well to the cell culture medium.

The next day, whether gene transfer was achieved was confirmed by measuring the enzyme activity (relative value) of luciferase protein produced from the pCMV-GL3 plasmid by using ONE-Glo Luciferase Assay System (Promega).

**Table 9**

| | Luciferase gene expression level (relative value) | | | |
|---|---|---|---|---|
| | Sorbitan sesquioleate | | | |
| | Sericin | FuGENE HD | Lipofectamine LTX&PLUS | Polyethylenimine Max |
| | Protamine sulfate | | | |
| Without Compound A | 12,768 | 9,732 | 8,309 | 113 |
| With Compound A | 184,829 | 276,812 | 157,534 | 2,017 |

As shown in Table 9, when compound A was used in combination with the composition of the present invention or a commercially available gene transfer reagent, i.e., Lipofectamine LTX&PLUS reagent, FuGENE HD transfection reagent, or Polyethylenimine Max, improvement in gene expression was confirmed in each case.

## Claims

1. A composition for gene transfer, the composition comprising:
(A) at least one lipid selected from the group consisting of sorbitan sesquioleate, sorbitan monolaurate, sorbitan monopalmitate, sorbitan monostearate, sorbitan trioleate, sorbitan monooleate, L-α-phosphatidylinositol, L-α-dioleoyl phosphatidylethanolamine, octadecylamine, hexadecylamine, DOTAP, and cardiolipin;
(B) at least one protein selected from the group consisting of albumin, casein, gelatin, and sericin; and
(C) at least one positively charged substance selected from the group consisting of protamine sulfate, polyarginine, polylysine, polyethyleneimine, and hexadimethrine bromide.

2. The composition according to claim 1, further comprising a gene.

3. The composition according to Claim 1 or 2, further comprising (D) at least one extracellular matrix component selected from the group consisting of laminin, fibronectin, vitronectin, hyaluronic acid, entactin, elastin, tenascin, collagen, chondroitin sulfate, fibrin, and fibrinogen.

4. The composition according to any one of claims 1 to 3, further comprising (E) at least one member selected from the group consisting of chloroquine, quinacrine, hydroxychloroquine, cyclosporin, cyclophosphamide, tacrolimus, ascomycin, rapamycin, 2-cyano-3-(3,4-dihydroxyphenyl)-N-benzylacrylamide, amlexanox, N-[3-[[5-iodo-4-[[3-[(2-thienylcarbonyl)amino]propyl]amino]-2-pyrimidinyl]amino]phenyl]-1-pyrrolidinecarboxamide, doxorubicin, actinomycin D, aurintricarboxylic acid, paclitaxel, etoposide, N-[1-(3-(5-chloro-3-methylbenzo[b]thiophen-2-yl)-1-methyl-1H-pyrazol-5-yl)]-2-chlorobenzenesulfonamide, α-amanitin, β-amanitin, amlodipine, nifedipine, nicardipine, verapamil, diltiazem, trichostatin A, tubastatin A, suberoyl bis-hydroxamic acid, suberoylanilide hydroxamic acid, valproic acid, and phorbol 12-myristate 13-acetate.

5. The composition according to any one of Claims 1 to 4, further comprising (F) a viral envelope.

6. The composition according to Claim 1, wherein the lipid of (A) is at least one member selected from the group consisting of sorbitan sesquioleate and sorbitan monolaurate.

7. The composition according to Claim 1, wherein the lipid of (A) is sorbitan sesquioleate.

8. A composition for use in introducing a gene into a cell of a non-human animal in in vivo therapy, the composition comprising:
(A) at least one lipid selected from the group consisting of sorbitan sesquioleate, sorbitan monolaurate, sorbitan monopalmitate, sorbitan monostearate, sorbitan trioleate, sorbitan monooleate, L-α-phosphatidylinositol, L-α-dioleoyl phosphatidylethanolamine, octadecylamine, hexadecylamine, DOTAP, and cardiolipin;
(B) at least one protein selected from the group consisting of albumin, casein, gelatin, and sericin; and
(C) at least one positively charged substance selected from the group consisting of protamine sulfate, polyarginine, polylysine, polyethyleneimine, and hexadimethrine bromide.

9. A method for introducing a gene into a cell in vitro, the method comprising bringing, into contact with the cell,
(A) at least one lipid selected from the group consisting of sorbitan sesquioleate, sorbitan monolaurate, sorbitan monopalmitate, sorbitan monostearate, sorbitan trioleate, sorbitan monooleate, L-α-phosphatidylinositol, L-α-dioleoyl phosphatidylethanolamine, octadecylamine, hexadecylamine, DOTAP, and cardiolipin;
(B) at least one protein selected from the group consisting of albumin, casein, gelatin, and sericin; and
(C) at least one positively charged substance selected from the group consisting of protamine sulfate, polyarginine, polylysine, polyethyleneimine, and hexadimethrine bromide; and the gene,
wherein the lipid, the protein, the positively charged substance, and the gene are all brought into contact with the cell simultaneously.

10. A kit for gene transfer, the kit comprising:
(A) at least one lipid selected from the group consisting of sorbitan sesquioleate, sorbitan monolaurate, sorbitan monopalmitate, sorbitan monostearate, sorbitan trioleate, sorbitan monooleate, L-α-phosphatidylinositol, L-α-dioleoyl phosphatidylethanolamine, octadecylamine, hexadecylamine, DOTAP, and cardiolipin;
(B) at least one protein selected from the group consisting of albumin, casein, gelatin, and sericin; and
(C) at least one positively charged substance selected from the group consisting of protamine sulfate, polyarginine, polylysine, polyethyleneimine, and hexadimethrine bromide.

## Patentansprüche

1. Zusammensetzung für Gentransfer, wobei die Zusammensetzung umfasst:
(A) mindestens ein Lipid, das ausgewählt ist aus der Gruppe, bestehend aus Sorbitansesquioleat, Sorbitanmonolaurat, Sorbitanmonopalmitat, Sorbitanmonostearat, Sorbitantrioleat, Sorbitanmonooleat, L-α-Phosphatidylinositol, L-α-Dioleoylphosphatidylethanolamin, Octadecylamin, Hexadecylamin, DOTAP und Cardiolipin;
(B) mindestens ein Protein, das ausgewählt ist aus der Gruppe, bestehend aus Albumin, Casein, Gelatine und Sericin; und
(C) mindestens eine positiv geladene Substanz, die ausgewählt ist aus der Gruppe, bestehend aus Protaminsulfat, Polyarginin, Polylysin, Polyethylenimin und Hexadimethrinbromid.

2. Zusammensetzung nach Anspruch 1, ferner umfassend ein Gen.

3. Zusammensetzung nach Anspruch 1 oder 2, ferner umfassend (D) mindestens eine extrazelluläre Matrixkomponente, die ausgewählt ist aus der Gruppe, bestehend aus Laminin, Fibronectin, Vitronectin, Hyaluronsäure, Entactin, Elastin, Tenascin, Collagen, Chondroitinsulfat, Fibrin und Fibrinogen.

4. Zusammensetzung nach einem der Ansprüche 1 bis 3, ferner umfassend (E) mindestens einen Vertreter, der ausgewählt ist aus der Gruppe bestehend, aus Chloroquin, Chinacrin, Hydroxychloroquin, Cyclosporin, Cyclophosphamid, Tacrolimus, Ascomycin, Rapamycin, 2-Cyano-3-(3,4-dihydroxyphenyl)-N-benzylacrylamid, Amlexanox, N-[3-[[5-Iod-4-[[3-[(2-thienylcarbonyl)amino]propyl]amino]-2-pyrimidinyl]amino]phenyl]-1-pyrrolidin-carbonsäureamid, Doxorubicin, Actinomycin D, Aurintricarbonsäure, Paclitaxel, Etoposid, N-[1-(3-(5-Chlor-3-methylbenzo[b]thiophen-2-yl)-1-methyl-1H-pyrazol-5-yl)]-2-chlorbenzolsulfonamid, α-Amanitin, β-Amanitin, Amlodipin, Nifedipin, Nicardipin, Verapamil, Diltiazem, Trichostatin A, Tubastatin A, Suberoylbishydroxamsäure, Suberoylanilid-hydroxamsäure, Valproinsäure und Phorbol-12-myristat-13-acetat.

5. Zusammensetzung nach einem der Ansprüche 1 bis 4, ferner umfassend (F) eine Virushülle.

6. Zusammensetzung nach Anspruch 1, wobei das Lipid von (A) mindestens ein Vertreter ist, der ausgewählt ist aus der Gruppe, bestehend aus Sorbitansesquioleat und Sorbitanmonolaurat.

7. Zusammensetzung nach Anspruch 1, wobei das Lipid von (A) Sorbitansesquioleat ist.

8. Zusammensetzung zur Verwendung zum Einführung eines Gens in eine Zelle eines nicht-humanen Lebewesens in einer In-vivo-Therapie, wobei die Zusammensetzung umfasst:
(A) mindestens ein Lipid, das ausgewählt ist aus der Gruppe, bestehend aus Sorbitansesquioleat, Sorbitanmonolaurat, Sorbitanmonopalmitat, Sorbitanmonostearat, Sorbitantrioleat, Sorbitanmonooleat, L-α-Phosphatidylinositol, L-α-Dioleoylphosphatidylethanolamin, Octadecylamin, Hexadecylamin, DOTAP und Cardiolipin;
(B) mindestens ein Protein, das ausgewählt ist aus der Gruppe, bestehend aus Albumin, Casein, Gelatine und Sericin; und
(C) mindestens eine positiv geladene Substanz, die ausgewählt ist aus der Gruppe, bestehend aus Protaminsulfat, Polyarginin, Polylysin, Polyethylenimin und Hexadimethrinbromid.

9. Verfahren zum Einführen eines Gens in eine Zelle in vitro, wobei das Verfahren das Inkontaktbringen der Zelle mit
(A) mindestens einem Lipid, das ausgewählt ist aus der Gruppe, bestehend aus Sorbitansesquioleat, Sorbitanmonolaurat, Sorbitanmonopalmitat, Sorbitanmonostearat, Sorbitantrioleat, Sorbitanmonooleat, L-α-Phosphatidylinositol, L-α-Dioleoylphosphatidylethanolamin, Octadecylamin, Hexadecylamin, DOTAP und Cardiolipin;
(B) mindestens einem Protein, das ausgewählt ist aus der Gruppe, bestehend aus Albumin, Casein, Gelatine und Sericin; und
(C) mindestens einer positiv geladenen Substanz, die ausgewählt ist aus der Gruppe, bestehend aus Protaminsulfat, Polyarginin, Polylysin, Polyethylenimin und Hexadimethrinbromid; und
dem Gen umfasst;
wobei das Lipid, das Protein, die positiv geladene Substanz und das Gen alle gleichzeitig mit der Zelle in Kontakt gebracht werden.

10. Kit für Gentransfer, wobei das Kit umfasst:
(A) mindestens ein Lipid, das ausgewählt ist aus der Gruppe, bestehend aus Sorbitansesquioleat, Sorbitanmonolaurat, Sorbitanmonopalmitat, Sorbitanmonostearat, Sorbitantrioleat, Sorbitanmonooleat, L-α-Phosphatidylinositol, L-α-Dioleoylphosphatidylethanolamin, Octadecylamin, Hexadecylamin, DOTAP und Cardiolipin;
(B) mindestens ein Protein, das ausgewählt ist aus der Gruppe bestehend aus Albumin, Casein, Gelatine und Sericin; und
(C) mindestens eine positiv geladene Substanz, die ausgewählt ist aus der Gruppe bestehend aus Protaminsulfat, Polyarginin, Polylysin, Polyethylenimin und Hexadimethrinbromid.

## Revendications

1. Composition pour le transfert d'un gène, la composition comprenant:
(A) au moins un lipide choisi dans le groupe constitué de: sesquioléate de sorbitan, monolaurate de sorbitan, monopalmitate de sorbitan, monostéarate de sorbitan, trioléate de sorbitan, monooléate de sorbitan, L-α-phosphatidylinositol, phosphatidyléthanolamine de L-α-dioléyle, octadécylamine, hexadécylamine, DOTAP et cardiolipine;
(B) au moins une protéine choisie dans le groupe constitué de: albumine, caséine, gélatine et séricine; et
(C) au moins une substance chargée positivement choisie dans le groupe constitué de: sulfate de protamine, polyarginine, polylysine, polyéthylèneimine et bromure d'hexadiméthrine.

2. Composition selon la revendication 1, comprenant en outre un gène.

3. Composition selon la revendication 1 ou 2, comprenant en outre (D) au moins un composant de matrice extracellulaire choisi dans le groupe constitué de: laminine, fibronectine, vitronectine, acide hyaluronique, entactine, élastine, ténascine, collagène, sulfate de chondroïtine, fibrine et fibrinogène.

4. Composition selon l'une quelconque des revendications 1 à 3, comprenant en outre (E) au moins un élément choisi dans le groupe constitué de: chloroquine, quinacrine, hydroxychloroquine, cyclosporine, cyclophosphamide, tacrolimus, ascomycine, rapamycine, 2-cyano-3-(3,4-dihydroxyphényl)-N-benzylacrylamide, amlexanox, N-[3-[[5-iodo-4-[[3-[(2-thiénylcarbonyl)amino]propyl]amino]-2-pyrimidinyl]amino]phényl]-1-pyrrolidinecarboxamide, doxorubicine, actinomycine D, acide aurintricarboxylique, paclitaxel, étoposide, N-[1-(3-(5-chloro-3-méthylbenzo[b]thiophén-2-yl)-1-méthyl-1H-pyrazol-5-yl)]-2-chlorobenzènesulfonamide, α-amanitine, β-amanitine, amlodipine, nifédipine, nicardipine, vérapamil, diltiazem, trichostatine A, tubastatine A, acide subéroyl-bis-hydroxamique, acide subéroylanilide hydroxamique, acide valproïque et phorbol 12-myristate 13-acétate.

5. Composition selon l'une quelconque des revendications 1 à 4, comprenant en outre (E) une enveloppe virale.

6. Composition selon la revendication 1, dans laquelle le lipide de (A) est au moins un élément choisi dans le groupe constitué de: sesquioléate de sorbitan et monolaurate de sorbitan.

7. Composition selon la revendication 1, dans laquelle le lipide de (A) est le sesquioléate de sorbitan.

8. Composition pour une utilisation dans l'introduction d'un gène dans une cellule d'un animal non humain dans une thérapie in vivo, la composition comprenant:
(A) au moins un lipide choisi dans le groupe constitué de: sesquioléate de sorbitan, monolaurate de sorbitan, monopalmitate de sorbitan, monostéarate de sorbitan, trioléate de sorbitan, monooléate de sorbitan, L-α-phosphatidylinositol, phosphatidyléthanolamine de L-α-dioléyle, octadécylamine, hexadécylamine, DOTAP et cardiolipine;
(B) au moins une protéine choisie dans le groupe constitué de: albumine, caséine, gélatine et séricine; et
(C) au moins une substance chargée positivement choisie dans le groupe constitué de: sulfate de protamine, polyarginine, polylysine, polyéthylèneimine et bromure d'hexadiméthrine.

9. Méthode pour l'introduction d'un gène dans une cellule in vitro, la méthode consistant à mettre en contact avec la cellule,
(A) au moins un lipide choisi dans le groupe constitué de: sesquioléate de sorbitan, monolaurate de sorbitan, monopalmitate de sorbitan, monostéarate de sorbitan, trioléate de sorbitan, monooléate de sorbitan, L-α-phosphatidylinositol, phosphatidyléthanolamine de L-α-dioléyle, octadécylamine, hexadécylamine, DOTAP et cardiolipine;
(B) au moins une protéine choisie dans le groupe constitué de: albumine, caséine, gélatine et séricine; et
(C) au moins une substance chargée positivement choisie dans le groupe constitué de: sulfate de protamine, polyarginine, polylysine, polyéthylèneimine et bromure d'hexadiméthrine;
et le gène,
dans laquelle le lipide, la protéine, la substance chargée positivement et le gène sont tous mis en contact avec la cellule simultanément.

10. Kit pour le transfert d'un gène, le kit comprenant:
(A) au moins un lipide choisi dans le groupe constitué de: sesquioléate de sorbitan, monolaurate de sorbitan, monopalmitate de sorbitan, monostéarate de sorbitan, trioléate de sorbitan, monooléate de sorbitan, L-α-phosphatidylinositol, phosphatidyléthanolamine de L-α-dioléyle, octadécylamine, hexadécylamine, DOTAP et cardiolipine;
(B) au moins une protéine choisie dans le groupe constitué de: albumine, caséine, gélatine et séricine; et
(C) au moins une substance chargée positivement choisie dans le groupe constitué de: sulfate de protamine, polyarginine, polylysine, polyéthylèneimine et bromure d'hexadiméthrine.
